# EUROPÄISCHE PATENTSCHRIFT

(11) **EP 1 943 968 B1**
(45) Veröffentlichungstag und Bekanntmachung des Hinweises auf die Patenterteilung: **05.10.2011**
(21) Anmeldenummer: 07121962.0
(22) Anmeldetag: 30.11.2007
(51) Int. Cl.: A61B 17/28, A61B 18/14

(54) **Chirurgisches Rohrschaftinstrument**
Tubular surgical instrument
Instrument chirurgical à tige tubulaire

(30) Priorität: 11.01.2007 DE 102007002250
(43) Veröffentlichungstag der Anmeldung: 16.07.2008
(73) Patentinhaber: Aesculap AG, 78532 Tuttlingen (DE)
(72) Erfinder: Kupferschmid, Bernhard, 78576, Emmingen-Liptingen (DE); Mayenberger, Rupert, 78239, Rielasingen (DE)
(74) Vertreter: Hoeger, Stellrecht & Partner Patentanwälte

(56) Entgegenhaltungen:
- EP-A- 1 177 771
- WO-A-93/12722
- US-A- 5 695 521
- US-A1- 2006 184 198

## Beschreibung

Die Erfindung betrifft ein chirurgisches Rohrschaftinstrument mit einem länglichen Rohrschaft und mit zwei am distalen Ende des Rohrschafts im Abstand nebeneinander angeordneten und elastisch auseinander biegbaren Lagerarmen, zwischen denen mindestens ein um eine quer zur Längsachse des Rohrschaftes verlaufende Drehachse verschwenkbares Werkzeug gelagert ist, und mit einer den Rohrschaft und die Lagerarme übergreifenden rohrförmigen Hülse.

Chirurgische Rohrschaftinstrumente dieser Art werden insbesondere bei minimalinvasiven Operationen eingesetzt, bei denen diese Instrumente durch rohrförmige Trokare in das Körperinnere eingeführt werden. Dazu ist es notwendig, dass der Außendurchmesser klein ist, trotzdem müssen die Instrumente stabil genug sein, um relativ große Kräfte zu übertragen.

Bei bekannten Rohrschaftinstrumenten dieser Art werden die Werkzeuge, beispielsweise Klemmbacken oder Schneidklingen, im Spalt zwischen den nebeneinander angeordneten Lagerarmen verschwenkbar gelagert, dazu wird der Spalt zwischen den Lagerarmen von einer Lagerwelle durchsetzt, auf der das Werkzeug oder die Werkzeuge gelagert sind. Die Werkzeuge können durch einen geeigneten Mechanismus, der von der proximalen Seite des Rohrschaftinstrumentes aus zu bedienen ist, um die Lagerwelle verschwenkt werden, und bei dieser Verschwenkbewegung können Kräfte von den Werkzeugen auf die Lagerarme ausgeübt werden, die in Richtung der Lagerwelle verlaufen und dabei die Lagerarme auseinander biegen, d.h. der Spalt zwischen den Lagerarmen wird dadurch aufgespreizt. Dies kann beispielsweise dann der Fall sein, wenn die Werkzeuge als Schneidklingen ausgebildet und so gegeneinander gebogen oder geschränkt sind, dass sie während des gesamten Schneidvorganges mit den Schneidkanten kräftig gegeneinander gedrückt werden. Dies führt zu einer elastischen Aufbiegung der Schneidklingen und/oder der Lagerarme.

An sich ist eine solche geringfügige Aufbiegung der Lagerarme unschädlich, es ergeben sich aber dann Schwierigkeiten, wenn das Rohrschaftinstrument von einer rohrförmigen Hülse überfangen ist, beispielsweise in Form eines dünnen Kunststoffschlauches zur Isolation und zur Verhinderung von Verletzungen. Diese Hülse überfängt üblicherweise die Lagerarme und wird beim elastischen Aufspreizen der Lagerarme gedehnt, so dass sie trichterförmig verformt wird. Dies kann das Einführen in den Trokar behindern und sollte daher vermieden werden.EP-1177771 offenbart ein Instrument mit den Merkmalen des Oberbegriffs des Anspruchs 1.

Es ist Aufgabe der Erfindung, ein chirurgisches Rohrschaftinstrument der gattungsgemäßen Art so auszubilden, dass ein unbeabsichtigtes Aufweiten der rohrförmigen Hülse auf dem Rohrschaftinstrument vermieden wird.

Diese Aufgabe wird bei einem chirurgischen Rohrschaftinstrument der eingangs beschriebenen Art erfindungsgemäß dadurch gelöst, dass der Abstand der Außenflächen der unverbogenen Lagerarme vom Rohrschaft zu den freien Enden der Lagerarme hin abnimmt, und dass die Außenflächen der unverbogenen Lagerarme im wesentlichen kegelmantelförmig ausgebildet sind.

Bei einer solchen Ausgestaltung ergibt sich zwischen den Außenflächen der unverbogenen Lagerarme und der Innenfläche der rohrförmigen Hülse ein Spalt, der vom Rohrschaft zum freien Ende der Lagerarme hin größer wird. Wenn die Lagerarme in der erörterten Weise bei der Verschwenkung der Werkzeuge elastisch nach außen gebogen werden, kann die Außenfläche diesen zum freien Ende hin größer werdenden Spalt verkleinern, ohne bei dieser Verbiegung an der Innenwand der rohrförmigen Hülse anzuliegen, d.h. eine Verformung der rohrförmigen Hülse unterbleibt.

Beispielsweise kann bei einem Rohrschaftinstrument mit einem kreiszylindrischen Querschnitt vorgesehen sein, dass die Außenflächen der unverbogenen Lagerarme im Wesentlichen kegelmantelförmig ausgebildet sind, damit ergibt sich eine konische Ausgestaltung der Lagerarme am distalen Ende des Schaftes.

Die Lagerarme können Teil des Rohrschaftes selbst sein, es ist aber bei einer abgewandelten Ausführungsform auch möglich, dass die Lagerarme Teil eines Lagerelementes sind, welches in den Rohrschaft eingesetzt ist. Dabei geht dieses Lagerelement an seiner Außenseite vorzugsweise stufenlos in den Rohrschaft über.

Die rohrförmige Hülse kann aus Metall oder Kunststoff bestehen, günstig ist es, wenn es sich dabei um einen Kunststoffschlauch handelt, insbesondere um einen Schrumpfschlauch.

Bei einer weiteren bevorzugten Ausführungsform ist zusätzlich vorgesehen, dass das Werkzeug oder gegebenenfalls die Werkzeuge an ihrer außen liegenden Kante eine Einkerbung aufweisen, in die bei schräg zur Längsachse des Rohrschaftes stehendem Werkzeug der distale Rand der rohrförmigen Hülse eintaucht. Auf diese Weise ist auch bei einem großen Öffnungswinkel sichergestellt, dass das geöffnete Werkzeug nicht am distalen Rand der rohrförmigen Hülse anschlägt und diese unbeabsichtigt aufweitet. Auch diese Maßnahme trägt also dazu bei, die unerwünschte Aufweitung der rohrförmigen Hülse zu vermeiden. Es kann auch vorgesehen sein, dass die Öffnungsbewegung der Werkzeuge begrenzt wird, beispielsweise durch geeignete Anschläge. Dadurch ist sichergestellt, dass die geöffneten Werkzeuge nicht am distalen Rand der rohrförmigen Hülse anschlagen und diese unbeabsichtigt aufweiten.

Besonders vorteilhaft ist die beschriebene Ausgestaltung bei einem Instrument, bei dem zwei als Schneidklingen ausgebildete Werkzeuge vorgesehen sind, die durch die Lagerarme in Richtung der Drehachse gegeneinander gedrückt werden. Die Lagerarme wirken somit als federndes Andruckelement, dadurch werden die Schneidklingen im Kontaktbereich gegeneinander gedrückt.

Insbesondere kann dabei vorgesehen sein, dass die Schneidklingen so gegeneinander gebogen sind, dass die Schneidklingen beim Schließen im Bereich ihrer Lagerung an den Lagerarmen mit einer ansteigenden Kraft auseinandergedrückt werden. Trotz dieser von den Lagerarmen aufzubringenden Kräfte und der daraus resultierenden Aufspreizung des Spaltes zwischen den Lagerarmen ist durch den abnehmenden Abstand der Außenflächen der Lagerarme sichergestellt, dass die Lagerarme die rohrförmige Hülse nicht aufweiten, sondern auch bei der Verbiegung mit ihrer Außenkontur immer innerhalb des Innenquerschnittes der rohrförmigen, unverformten Hülse verbleiben.

Die nachfolgende Beschreibung bevorzugter Ausführungsformen der Erfindung dient im Zusammenhang mit der Zeichnung der näheren Erläuterung. Es zeigen:

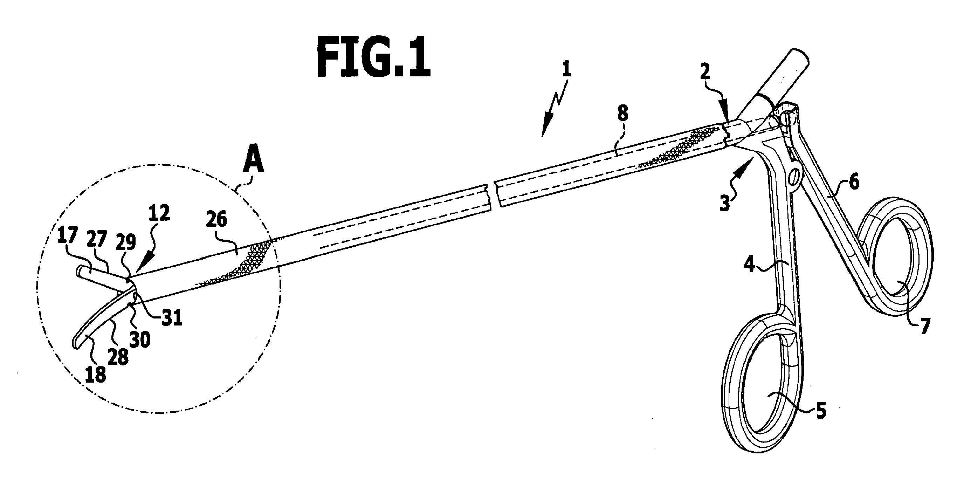

- Figur 1:: eine perspektivische Ansicht eines Rohrschaftinstrumentes;
- Figur 2:: eine vergrößerte Längsschnittansicht des Bereiches A in Figur 1 mit den Werkzeugen in einer Öffnungsstellung;
- Figur 2a:: eine vergrößerte Detailansicht des Bereiches C in Figur 2;
- Figur 3:: eine Draufsicht auf den distalen Bereich des Rohrschaftinstrumentes in Richtung des Pfeiles B in Figur 2;
- Figur 4:: eine Ansicht ähnlich Figur 2 mit den Werkzeugen in Schließstellung und
- Figur 5:: eine Ansicht ähnlich Figur 3 mit den Werkzeugen in Schließstellung.

Das in der Zeichnung dargestellte Rohrschaftinstrument 1 umfasst einen länglichen, rohrförmigen Schaft 2, an dessen proximalem Ende 3 eine etwa quer von der Längsrichtung des Rohrschaftinstrumentes 1 abstehende Branche 4 mit einer Fingeröffnung 5 angesetzt ist. Eine zweite Branche 6 mit einer Fingeröffnung 7 ist um eine quer zur Längsachse des Rohrschaftinstrumentes 1 verlaufende Schwenkachse verschwenkbar mit der feststehenden Branche 4 verbunden, so dass die beiden Branchen 4, 6 gegeneinander und wieder auseinander geschwenkt werden können.

Im Inneren des Schaftes 2 ist eine in Längsrichtung des Schaftes 2 frei verschiebbare Schub- und Zugstange 8 gelagert, die am proximalen Ende gelenkig mit der verschwenkbaren Branche 6 verbunden ist, so dass bei deren Verschwenkbewegung die Schub- und Zugstange 8 im Schaft 2 vor- bzw. zurückgeschoben wird.

Am distalen Ende trägt die Schub- und Zugstange 8 einen Gleitstein 9 mit gebogenen, rinnenförmigen Vertiefungen 10, 11 auf gegenüberliegenden Seiten.

Am distalen Ende 12 trägt der Schaft 2 zwei in Längsrichtung des Schaftes parallel und mit Abstand zueinander verlaufende Lagerarme 13, 14, die zwischen sich einen Lagerspalt 15 ausbilden. Dieser wird von einer an den beiden Lagerarmen 13, 14 gehaltenen, quer zur Längsrichtung des Schaftes 2 verlaufenden Lagerwelle 16 überbrückt, die im vorderen Bereich der Lagerarme 13, 14 nahe dem freien Ende der Lagerarme 13, 14 angeordnet ist.

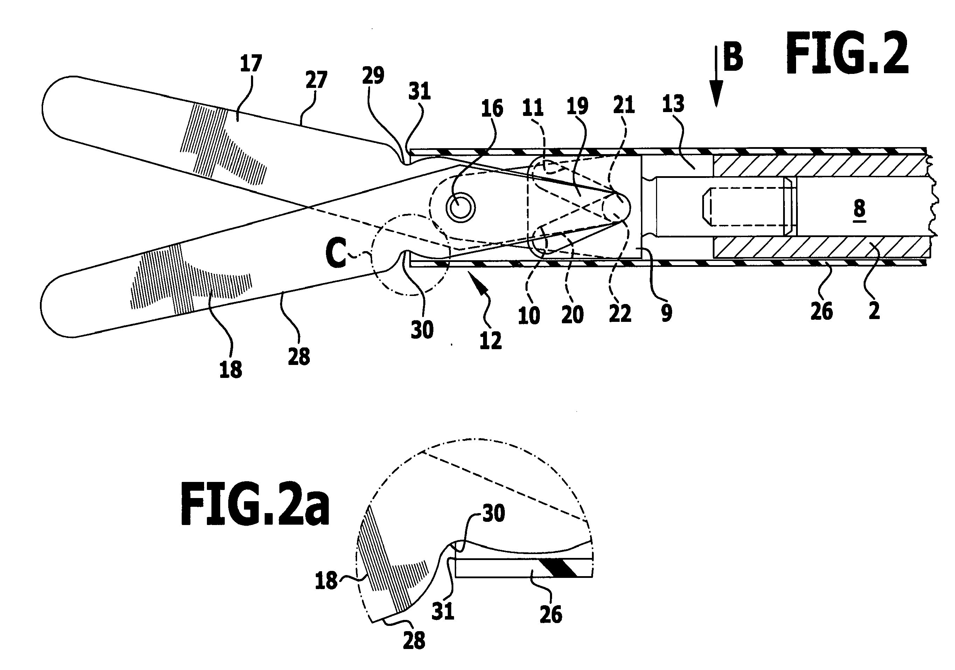

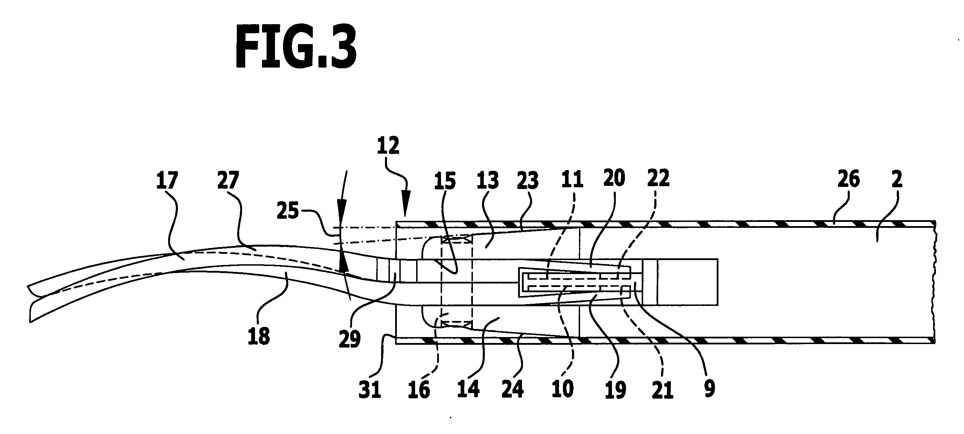

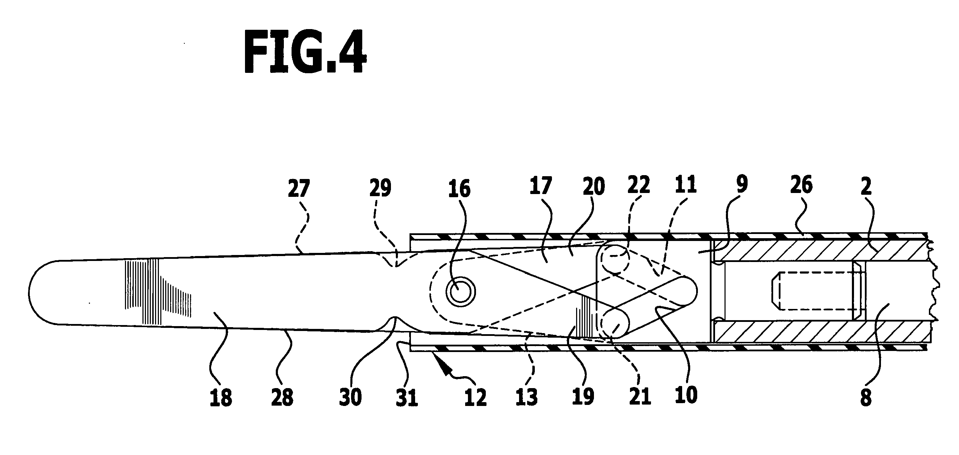

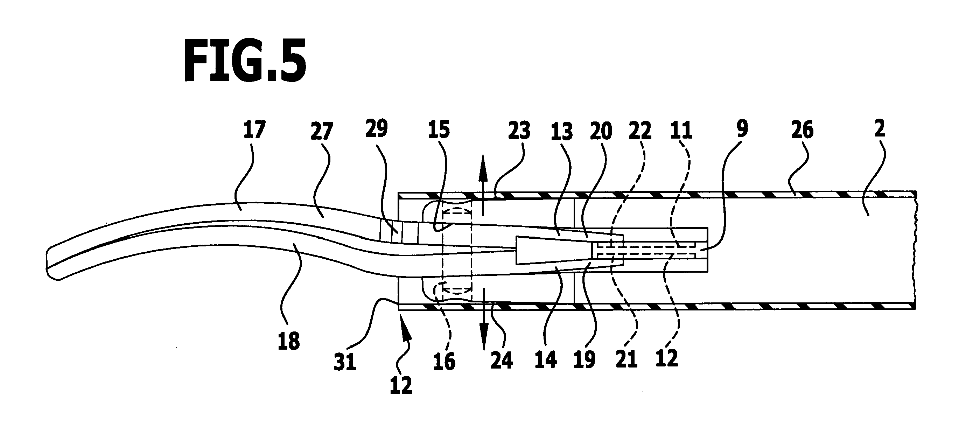

Auf der Lagerwelle 16 sind zwei Schneidklingen 17, 18 um die durch die Lagerwelle 16 gebildete Drehachse verschwenkbar gelagert. Eine Verschwenkung erfolgt durch eine Längsverschiebung des Gleitsteines 9, der sich am proximalen Ende des Lagerspaltes 15 in diesem befindet und an dessen Seitenwänden die rückwärtigen Enden 19, 20 der Schneidklingen 17, 18 anliegen. In diesem Anlagebereich tragen die rückwärtigen Enden 19, 20 seitlich vorstehende Zapfen 21, 22, die in die rinnenförmigen Vertiefungen 10 bzw. 11 des Gleitsteines 9 eintauchen und bei Längsverschiebung des Gleitsteines 9 durch die Seitenwände dieser rinnenförmigen Vertiefungen 10, 11 geführt werden. Diese Bewegung wird in eine Verschwenkbewegung der Schneidklingen 17, 18 übersetzt, so dass diese bei zurückgezogenem Gleitstein 9 geschlossen sind (Figuren 4 und 5), bei vorgeschobenem Gleitstein 9 dagegen geöffnet (Figuren 2 und 3).

Die beiden Schneidklingen 17, 18 sind in Längsrichtung seitlich abgebogen, und zwar derart, dass bei der Bewegung der Schneidklingen 17, 18 von der Öffnungsstellung in die Schließstellung der Kontaktbereich zwischen den beiden Schneidklingen 17, 18 in distaler Richtung verschoben wird. Die beiden Schneidklingen 17, 18 liegen dabei an den einander gegenüberliegenden, ebenen Innenseiten der Lagerarme 13, 14 an und werden dadurch gegeneinander gedrückt. Durch die Formgebung der Schneidklingen 17 und 18 steigt die Kraft an, mit der die Schneidklingen 17, 18 beim Schließen gegeneinander gedrückt werden, und dies führt dazu, dass die beiden Lagerarme 13, 14 elastisch nach außen gebogen werden, d.h. der Lagerspalt 15 wird aufgeweitet.

Aus diesem Grund sind die Außenflächen 23, 24 der Lagerarme 13, 14 konisch ausgebildet, d.h. sie liegen bei unverformten Lagerarmen auf einem Kegelmantel, wobei der Durchmesser vom proximalen zum distalen Ende hin abnimmt. Es entsteht dadurch bei unverbogenen Lagerarmen zwischen den Au-βenflächen 23 und 24 und einer der Außenfläche des Schaftes 2 entsprechenden Zylinderfläche ein Ringspalt 25 mit in distaler Richtung zunehmender Breite (Figur 3).

Beim elastischen Aufbiegen der Lagerarme 13, 14 in Längsrichtung der Lagerwelle 16 können die Außenflächen 23 und 24 in diesen Ringspalt 25 eintreten und verbleiben dabei trotzdem immer noch innerhalb des Querschnittes der durch die Außenseite des Schaftes 2 definierten Zylinderfläche. Es ist damit sichergestellt, dass die Außenflächen 23 und 24 bei der Schließbewegung der Schneidklingen 17, 18 nicht nach außen über diese Zylinderfläche hervortreten.

Auf den Schaft 2 ist eine zylinderförmige Hülse 26 aufgeschoben, die sich in distaler Richtung bis über die freien Enden der Lagerarme 13, 14 hin erstreckt und diese vollständig seitlich abdeckt. Diese zylinderförmige Hülse 26 wird auch bei elastisch nach außen gebogenen Lagerarmen 13, 14 von diesen nicht erreicht und nicht nach außen ausgeweitet.

Die Schneidklingen 17, 18 tragen an ihren Außenkanten 27, 28 in einem der Lagerwelle 16 naheliegenden Abschnitt jeweils eine Einkerbung 29 bzw. 30, die so angeordnet sind, dass beim Verschwenken der Schneidklingen 17, 18 in die Öffnungsstellung (Figur 2) der distale Rand 31 der Hülse 26 in diese Einkerbungen 29, 30 eintaucht, so dass die Schneidklingen 17, 18 in der Öffnungsstellung einen großen Winkel gegenüber der Längsachse des Schaftes 2 einnehmen können, ohne dadurch am distalen Rand 31 der Hülse 26 anzustoßen und diese aufzuweiten.

Die geschilderten Maßnahmen dienen jeweils für sich und auch gemeinsam dazu, eine unerwünschte Aufweitung der Hülse 26 zu vermeiden.

## Patentansprüche

1. Chirurgisches Rohrschaftinstrument (1) mit einem länglichen Rohrschaft (2) und mit zwei am distalen Ende (12) des Rohrschafts (2) im Abstand nebeneinander angeordneten und elastisch auseinander biegbaren Lagerarmen (13, 14), zwischen denen mindestens ein um eine quer zur Längsachse des Rohrschaftes (2) verlaufende Drehachse verschwenkbares Werkzeug gelagert ist, und mit einer den Rohrschaft (2) und die Lagerarme (13, 14) übergreifenden rohrförmigen Hülse (26), wobei der Abstand der Außenflächen (23, 24) der unverbogenen Lagerarme (13, 14) vom Rohrschaft (2) zu den freien Enden der Lagerarme (13, 14) hin abnimmt, **dadurch gekennzeichnet, dass** die Außenflächen (23, 24) der unverbogenen Lagerarme (13, 14) im wesentlichen kegelmantelförmig ausgebildet sind.

2. Rohrschaftinstrument nach Anspruch 1, **dadurch gekennzeichnet, dass** die Lagerarme (13, 14) Teil eines Lagerelements sind, welches in den Rohrschaft (2) eingesetzt ist.

3. Rohrschaftinstrument nach einem der Ansprüche 1 oder 2, **dadurch gekennzeichnet, dass** die rohrförmige Hülse (26) ein Kunststoffschlauch ist.

4. Rohrschaftinstrument nach einem der voranstehenden Ansprüche, **dadurch gekennzeichnet, dass** das Werkzeug oder gegebenenfalls die Werkzeuge (17, 18) an ihrer außen liegenden Kante (27, 28) eine Einkerbung (29, 30) aufweisen, in die bei schräg zur Längsachse des Rohrschaftes (2) stehendem Werkzeug (17, 18) der distale Rand (31) der rohrförmigen Hülse (26) eintaucht.

5. Rohrschaftinstrument nach einem der voranstehenden Ansprüche, **dadurch gekennzeichnet, dass** zwei als Schneidklingen ausgebildete Werkzeuge (17, 18) vorgesehen sind, die durch die Lagerarme (13, 14) in Richtung der Drehachse gegeneinander gedrückt werden.

6. Rohrschaftinstrument nach Anspruch 5, **dadurch gekennzeichnet, dass** die Werkzeuge (17, 18) so gegeneinander gebogen sind, dass sie beim Schließen im Bereich ihrer Lagerung an den Lagerarmen (13, 14) mit einer ansteigenden Kraft auseinandergedrückt werden.

## Claims

1. Tubular shafted surgical instrument (1) with an elongate tubular shaft (2) and with two bearing arms (13, 14), which are spaced adjacent to one another at the distal end (12) of the tubular shaft (2) and can be bent elastically apart and between which is mounted at least one tool, which can be pivoted around a rotational axis running transversely to the longitudinal axis of the tubular shaft (2), and with a tubular sleeve (26) engaging over the tubular shaft (2) and the bearing arms (13, 14), wherein the spacing of the outer faces (23, 24) of the non-bent bearing arms (13, 14) from the tubular shaft (2) decreases towards the free ends of the bearing arms (13, 14), **characterised in that** the outer faces (23, 24) of the non-bent bearing arms (13, 14) are configured with a substantially tapering shape.

2. Tubular shafted instrument according to claim 1, **characterised in that** the bearing arms (13, 14) are part of a bearing element, which is inserted into the tubular shaft (2).

3. Tubular shafted instrument according to one of claims 1 or 2, **characterised in that** the tubular sleeve (26) is a plastic tube.

4. Tubular shafted instrument according to one of the preceding claims, **characterised in that** the tool or tools (17, 18) have a notch (29, 30) on their outside edge (27, 28), into which notch the distal edge (31) of the tubular sleeve (26) penetrates when the tool (17, 18) extends at an angle to the longitudinal axis of the tubular shaft (2).

5. Tubular shafted instrument according to one of the preceding claims, **characterised in that** two tools (17, 18) configured as cutting blades are provided, which are pressed against one another in the direction of the rotational axis by the bearing arms (13, 14).

6. Tubular shafted instrument according to claim 5, **characterised in that** the tools (17, 18) are bent relative to one another in such a way that during closure they are pressed apart in the region of their mounting on the bearing arms (13, 14) with an increasing force.

## Revendications

1. Instrument chirurgical à tige tubulaire (1) avec une tige tubulaire allongée (2) et avec deux bras de support (13, 14) disposés côte à côte à distance à l'extrémité distale (12) de la tige tubulaire (2) et pliables élastiquement en s'écartant l'un de l'autre, entre lesquels est monté au moins un outil pivotable autour d'un axe de rotation qui s'étend transversalement par rapport à l'axe longitudinal de la tige tubulaire (2), et avec une gaine tubulaire (26) recouvrant la tige tubulaire (2) et les bras de support (13, 14), où la distance des surfaces externes (23, 24) des bras de support (13, 14) non pliés diminue de la tige tubulaire (2) aux extrémités libres des bras de support (13, 14), **caractérisé en ce que** les surfaces externes (23, 24) des bras de support (13, 14) non pliés sont sensiblement sous forme de surface conique.

2. Instrument à tige tubulaire selon la revendication 1, **caractérisé en ce que** les bras de support (13, 14) font partie d'un élément de support qui est inséré dans la tige tubulaire (2).

3. Instrument à tige tubulaire selon l'une des revendications 1 ou 2, **caractérisé en ce que** la gaine tubulaire (26) est un tuyau en matière synthétique.

4. Instrument à tige tubulaire selon l'une des revendications précédentes, **caractérisé en ce que** l'outil ou éventuellement les outils (17, 18) présentent au niveau de leur arête située à l'extérieur (27, 28) une rainure (29, 30) dans laquelle pénètre le bord distal (31) de la gaine tubulaire (26) lorsque l'outil (17, 18) est situé en biais par rapport à l'axe longitudinal de la tige tubulaire (2).

5. Instrument à tige tubulaire selon l'une des revendications précédentes, **caractérisé en ce que** deux outils (17, 18) en forme de lames de coupe, qui sont poussés l'un contre l'autre par les bras de support (13, 14) en direction de l'axe de rotation, sont prévus.

6. Instrument à tige tubulaire selon la revendication 5, **caractérisé en ce que** les outils (17, 18) sont pliés l'un contre l'autre de telle manière que, lors de la fermeture, ils sont poussés en s'écartant l'un de l'autre avec une force croissante dans le domaine de leur fixation sur les bras de support (13, 14).
